# EUROPEAN PATENT SPECIFICATION

(11) **EP 3 068 448 B1**
(45) Date of publication and mention of the grant of the patent: **02.10.2019**
(21) Application number: 14799852.0
(22) Date of filing: 11.11.2014
(51) Int. Cl.: A61L 15/42, A61L 15/60, A61L 15/28

(54) **MULTILAYER COMPOSITION**
MEHRSCHICHTIGE ZUSAMMENSETZUNG
COMPOSITION MULTICOUCHE

(30) Priority: 12.11.2013 GB 201319936
(43) Date of publication of application: 21.09.2016
(73) Proprietor: First Water Limited, Marlborough Wiltshire SN8 2RB (GB)
(72) Inventor: ANDREWS, Philip, Marlborough Wiltshire SN8 2RB (GB)
(74) Representative: Haseltine Lake Kempner LLP
(86) International application number: PCT/GB2014/053342
(87) International publication number: WO 2015/071653

(56) References cited:
- WO-A1-2009/090403
- WO-A1-2010/109239
- WO-A2-2007/007115

## Description

The present invention is directed to a novel multilayer composition having an improved design that is suitable for the treatment or prevention of wounds. In particular, the present invention is directed to a novel multilayer composition that is suitable for use in the treatment or prevention of blisters.

Plasters for use in the treatment of blisters are known, and are used to shield and cushion blister hotspots helpingF reduce pain and aid healing or for application to areas known for rubbing to help reduce the chances of blister formation.

WO2010/109239 discloses a layered composition comprising a first layer comprising a gel material, a second layer containing a second material harder than the first material and a third layer comprising a low friction material e.g. polyurethane and having a thickness of 0.025 mm or more. One or more porous sheets , for example a scrim, may be disposed within or on a surface of one or more layer, and preferably within or on the surface the gel material, preferably a hydrogel material. Such a scrim may suitably be in the form of a net or a woven or non-woven fabric. Preferred scrims include those formed from polyolefins, polyamides, polyacrylates or polyesters.

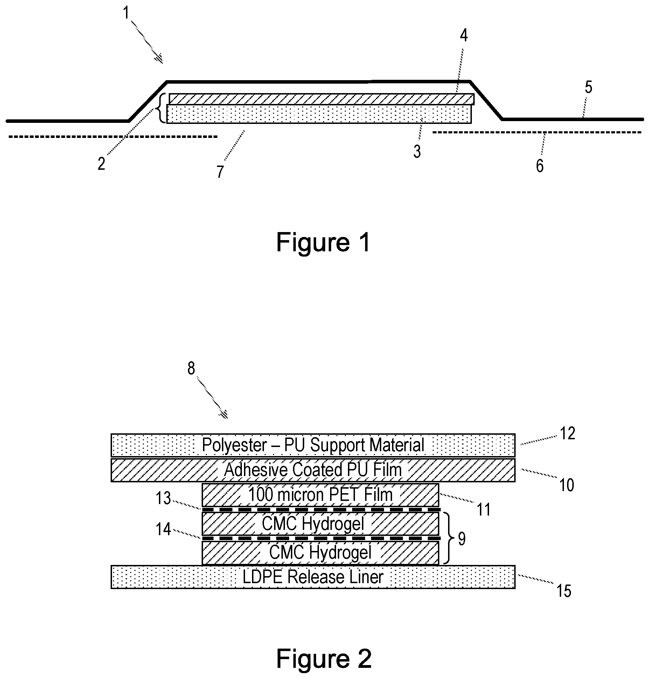

The structure of an existing plaster is shown in Figure 2 and comprises an area of a clear, adhesive hydrogel adhered centrally to a medical grade polyurethane (PU) film coated with a medical grade pressure sensitive adhesive. The upper surface of the dressing has a film to support the adhesive coated PU film which is made from polyethylene terephthalate (PET) and the lower surface has a release liner. There is also a polyester film between the hydrogel and the PU top film to create a shield that dissipates pressure from the footwear away from the blister.

The hydrogel layer contains two "scrim" layers embedded within it (represented by dotted black lines in figure 2) to add dimensional stability to this soft material.

In this design, the use of a material to form a shield layer on the back of the hydrogel island gives excellent performance in terms of spreading the applied force over the entire hydrogel area and thus helping "cushion" the user from point loading which could potentially cause discomfort. However, it has very limited flexibility, is non elastic and has a very low mean vapor transmission rate (< 200 g/m2/24h).

These physical properties result in the product being poor in terms of being able to take a compound curvature to form around the complex shapes of the foot and having limited breathability in the region where the shield layer is located.

The inability of the product to take compound curvature means the product tends to crease on application, giving a potential "weak spot" for failure of adhesion. The limited breathability could give rise to elevation of foot skin moisture levels in the area around the adhesion site, which is again believed to contribute to poor adhesion as acrylic adhesives are known to have reduced tack performance in the presence of moisture.

In addition to the problems with curvature and breathability of the shield layer, the rigidity of the PET material also manifests as a sharp, stepped edge on the back edge of the plaster when applied to the foot - that this sharp edge can catch on footwear or clothing causing the plaster to be pulled off the foot which causes considerable discomfort to a user/blister sufferer.

Further disadvantages with this current design include users having difficulty in removing the backing layer from the plaster with the entire plaster coming of when trying to do so, and the PU adhesive layer surrounding the hydrogel island being too thin and creasing up on application.

The present invention as described in more detail below aims to overcome or mitigate the problems in the existing art described above.

According to a first aspect of the present invention there is provided a multilayer composition comprising:
a first layer comprising a first material which is at least partially impregnated with a second material wherein the first material is a gel material and the second material is a flexible porous material, and
a second layer comprising a low friction material wherein the composition is adapted so that the first layer is in fluid flow communication with a wound or skin of a wearer in use wherein the composition does not include an additional distinct/discrete layer between the first and second layers.

The first material comprises a hydrogel. A "gel" includes, but is not limited to, a self-supporting, flexible substance, optionally comprising one or more polymers. A gel may comprise water. The first material is preferably a viscoelastic hydrogel. The first layer may consist essentially of or consist of a hydrogel, and any water contained therein. "Consist essentially of" in this context includes, but is not limited to, the first layer comprising about 5wt% or less, preferably about 2 wt% or less, of components other than the gel and any water it may contain.

The first layer may have a thickness of about 0.5 - 1.6 mm. Preferably the thickness of the layer is 0.8mm - 1.2mm.

The coat weight of the hydrogel is between 900 - 1400g/m² (gsm). Preferably the coat weight of the hydrogel is 1100 g/m²_{.}

The gel in first layer comprises a hydrogel.

The gel may comprise, consist essentially of or consist of a cross-linked hydrophilic polymer of a hydrophilic monomer and optionally one or more comonomers, together with water and/or one or more organic plasticiser, and optionally together with one or more additives selected from surfactants, polymers, pH regulators, electrolytes, chloride sources, bioactive compounds (including antimicrobial agents) and mixtures thereof, with less than about 30% by weight of other additives.

The first layer comprises a hydrogel. The expression "hydrogel" and like expressions, used herein, are not to be considered as limited to gels which contain water, but extend generally to all hydrophilic gels, including those containing organic non-polymeric components in the absence of water. The gel forming agent may, for example, be selected from natural hydrophilic polymers, synthetic hydrophilic polymers, hydrocolloids, gelling hydrophilic biopolymers and all combinations thereof.

Hydrogels are, generally speaking, hydrophilic polymers characterized by their hydrophilicity (i.e capacity to absorb large amounts of fluid such as wound exudate) and insolubility in water: i.e. they are capable of swelling in water while generally preserving their shape.

The hydrophilicity is generally due to groups such as hydroxyl, carboxy, carboxamido, sulfonates, and esters, among others. On contact with water, the hydrogel assumes a swollen hydrated state that results from a balance between the dispersing forces acting on hydrated chains and cohesive forces that do not prevent the penetration of water into the polymer network. The cohesive forces are most often the result of crosslinking, but may result from electrostatic, hydrophobic or dipole-dipole interactions.

Useful classes of hydrogels in the present invention include those polymers and copolymers derived from acrylic and methacrylic acid ester, including hydroxyalkyl (meth)acrylates, 2-(N,N-dimethylamino)ethyl methacrylate, methacryloyloxyalkyl sulfonates (generally crosslinked with diacrylate or divinylbenzene), polymers and copolymers of substituted and unsubstituted acrylamides, polymers and copolymers of N-vinylpyrrolidinone, and polyelectrolyte complexes. Hydrogels are described in greater detail in Hydrogels, Kirk-Othmer Encyclopedia of Chemical Technology, 4th Edition, vol. 7, pp. 783-807, John Wiley and Sons, New York.

The term "hydrogel" is used herein regardless of the state of hydration.

The hydrogel used in connection with the present invention will suitably comprise a substantially water-insoluble, crosslinked, at least partially neutralized, gel-forming polymer material. Such hydrogel materials can be prepared from polymerisable, unsaturated, acid- and ester-containing monomers. Examples of such monomers are described in more detail below.

The hydrogel used in the present invention preferably comprises a plasticised three-dimensional matrix of cross-linked polymer molecules, and has sufficient structural integrity to be self-supporting even at very high levels of internal water content (e.g. a water content of at least 20%, optionally 50%, by weight) with sufficient flexibility to conform to the surface contours of mammalian skin or other surface with which it is in contact.

The hydrogel generally comprises, in addition to the cross-linked polymeric network, an aqueous or non-aqueous plasticising medium including an organic plasticiser. This plasticising medium is preferably present in the same precursor solution as the monomer(s).

The precursor liquid can comprise a solution of the gel-forming polymer in a relatively volatile solvent, whereby the hydrogel is deposited as a residue on evaporation of the solvent, or - more preferably - the precursor liquid will comprise a solution of the monomer(s), cross-linking agent, plasticiser, and optionally water and other ingredients as desired, whereby the hydrogel is formed by a curing reaction performed on the precursor liquid after application to the substrate to which the hydrogel is to be applied.

In the following discussion, the second form of precursor solution and application protocol (in situ polymerisation of the hydrogel) will be discussed. The solvent deposition method carried out on a pre-formed gel-forming polymer is well known and the details of that procedure do not need to be reproduced here.

The polymerisation reaction is preferably a free-radical polymerisation with crosslinking, which may for example be induced by light, heat, radiation (e.g. ionising radiation), or redox catalysts, as is well known.

For example, the free radical polymerisation may be initiated in known manner by light (photoinitiation), particularly ultraviolet light (UV photoinitiation); heat (thermal initiation); electron beam (e-beam initiation); ionising radiation, particularly gamma radiation (gamma initiation); non-ionising radiation, particularly microwave radiation (microwave initiation); or any combination thereof. The precursor solution may include appropriate substances (initiators), at appropriate levels, e.g. up to about 5% by weight, more particularly between about 0.002% and about 2% by weight, which serve to assist the polymerisation and its initiation, in generally known manner.

Preferred photoinitiators include any of the following either alone or in combination: Type 1-hydroxy-ketones and benzilidimethyl-ketals e.g. lrgacure 651. These are believed on irradiation to form benzoyl radicals that initiate polymerisation.

Photoinitiators of this type that are preferred are those that do not carry substituents in the para position of the aromatic ring. A particularly preferred photoinitiator is 1-hydroxycyclohexyl phenyl ketone; for example, as marketed under the trade name lrgacure 184 by Ciba Speciality Chemicals. Also preferred are Daracur 1173 (2-hydroxy-2-propyl phenyl ketone) and mixtures of lrgacure 184 and Daracur 1173.

Photo-polymerisation is particularly suitable, and may be achieved using light, optionally together with other initiators, such as heat and/or ionizing radiation.

Photoinitiation will usually be applied by subjecting the pre-gel reaction mixture containing an appropriate photoinitiation agent to ultraviolet (UV) light. The incident UV intensity, at a wavelength in the range from 240 to 420nm, is typically greater than about 10mW/cm².

The processing will generally be carried out in a controlled manner involving a precise predetermined sequence of mixing and thermal treatment or history.

The UV irradiation time scale should ideally be less than 60 seconds, and preferably less than 10 seconds to form a gel with better than 95% conversion of the monomers.

Those skilled in the art will appreciate that the extent of irradiation will be dependent on a number of factors, including the UV intensity, the type of UV source used, the photoinitiator quantum yield, the amount of monomer(s) present, the nature of the monomer(s) present and the presence of polymerisation inhibitor. In one preferred embodiment, (on the one hand) the precursor solution in contact with the substrate to which it is to be applied and (on the other hand) the source of the polymerisation initiator (e.g. the radiation source) may move relative to one another for the polymerisation step. In this way, a relatively large amount of polymerisable material can be polymerised in one procedure, more than could be handled in a static system. This moving, or continuous, production system is preferred. After completion of the polymerisation, the product is preferably sterilised in conventional manner. The sterile composite may be used immediately, e.g. to provide a skin-adhesive layer in an article, or a top release layer may be applied to the composite for storage and transportation of the composite. If desired, certain ingredients of the hydrogel may be added after the polymerisation and optional cross-linking reaction. However, it is generally preferred that substantially all of the final ingredients of the hydrogel are present in the precursor solution, and that - apart from minor conventional conditioning or, in some cases, subsequent modifications caused by the sterilisation procedure - substantially no chemical modification of the hydrogel takes place after completion of the polymerisation reaction.

### Monomers

The gel preferably comprises a polymer having pendant sulphonyl groups, and optionally pendant carboxylic acid groups in acid or salt form. The hydrogel used in the present invention suitably may comprise a substantially water-insoluble, crosslinked, at least partially neutralized, gel-forming polymer material having the pendant sulphonyl groups, and optionally pendant carboxylic groups, in acid or salt form at least at its wound-contacting surface. The hydrogel polymer materials can be prepared from polymerizable, unsaturated, acid- and ester-containing monomers. The hydrogel polymer may be present at the wound-contacting surface of the composition and contain pendant sulphonyl groups, in acid or salt form, and optionally carboxylic groups in acid or salt form. Thus, such monomers include the olefinically unsaturated acids, esters and anhydrides which contain at least one carbon to carbon olefinic double bond. More specifically, these monomers can be selected from olefinically unsaturated carboxylic acids, carboxylic esters, carboxylic acid anhydrides; olefinically unsaturated sulphonic acids; and mixtures thereof.

Olefinically unsaturated carboxylic acid, carboxylic acid ester and carboxylic acid anhydride monomers include the acrylic acids typified by acrylic acid itself, methacrylic acid, ethacrylic acid, a-chloroacrylic acid, a-cyano-acrylic acid, ∼-methyl-acrylic acid (crotonic acid), a-phenyl acrylic acid, ∼-acryloxy-propionic acid, sorbic acid, a-chlorosorbic acid, angelic acid, cinnamic acid, /7-chlorocinnamic acid, ∼-styryl-acrylic acid (1- carboxy-4-phenyl-1,3-butadiene), itaconic acid, citraconic acid, mesaconic acid, glutaconic acid, aconitic acid, maleic acid, fumaric acid, tricarboxy-ethylene and maleic acid anhydride and salts (e.g. alkali metal salts such as sodium, potassium and lithium salts) thereof. For forming any polymer to be present at the lesion- contacting surface of the composition, the monomer or monomer mixture will include a monomer containing pendant sulphonyl groups, e.g. -803" in acid or salt form.

Olefinically unsaturated sulphonic acid monomers include aliphatic or aromatic vinyl sulphonic acids such as vinylsulphonic acid, allylsulphonic acid, vinyltoluenesulphonic acid and styrene sulphonic acid; vinyl sulphobetaines such as SPDA (1-propanaminium N,N-dimethyi-N-[2-[(1-oxo-2-propenyl)oxy]-3-sulfo hydroxide, inner salt (available from Raschig); acrylic and methacrylic sulphonic acid such as sulphoethyl acrylate, sulphoethyl methacrylate, sulphopropyl acrylate, sulphopropyl methacrylate, 2-hydroxy-3-acryloxy propyl sulphonic acid, 2-hydroxy-3- methacryloxy propyl sulphonic acid and 2-acrylamido-2-methylpropanesulphonic acid and salts (e.g. ammonium or alkali metal salts, such as sodium, potassium and lithium salts, or alkaline earth metal salts, such as calcium or magnesium) thereof.

The monomers may suitably be used in admixture with each other or with other monomers. In one particularly useful embodiment of the invention, a monomer which has a first counter- cation associated with it may be used in admixture with one or more monomer which has/have one or more second/further counter-cation(s) associated with it/them. The first counter-cation and the second/further counter cation may be the same or different. The monomers in their anionic form (i.e. disregarding the counter-cation) may be the same or different. In this way, the proportions of different cations (e.g. alkali metal ions such as sodium or potassium, or ammonium ions) can be finely controlled in the resultant polymer (homopolymer or copolymer). The particular weight ratios of one monomer to the or each other monomer can be selected within wide limits by those skilled in the art, depending on the desired propoerties of the resultant hydrogel polymer.

Further examples of suitable monomers for use in the present invention include: a polyalkylene glycol acrylate or a substituted derivative thereof; a polyalkylene glycol methacrylate or a substituted derivative thereof; acrylic acid and salts thereof (e.g. alkali metal salts such as sodium, potassium and lithium salts); 2-acrylamido-2-methylpropanesulphonic acid and salts thereof (e.g. ammonium or alkali metal salts, such as sodium, potassium and lithium salts, or alkaline earth metal salts, such as calcium or magnesium); acrylic acid (3-sulphopropyl) ester or a substituted derivative thereof or a salt thereof (e.g. an alkali metal salt such as sodium, potassium or lithium salt); diacetone acrylamide (N-1,1-dimethyl-3-oxobutyl-acrylamide); a vinyl lactam (e.g. N-vinyl pyrrolidone or a substituted derivative thereof); an optionally substituted N-alkylated acrylamide such as hydroxyethyl acrylamide; and an optionally substituted N,N-dialkylated acrylamide; and/or N-acryloyl morpholine or a substituted derivative thereof. For forming any polymer of the hydrogel, which may in use contact the wound, the monomer or monomer mixture may include a monomer containing pendant sulphonyl groups, e.g. -S03- in acid or salt form, and optionally carboxylic groups in acid or salt form.

Particularly preferred monomers include: the sodium salt of 2-acrylamido-2-methylpropane sulphonic acid, commonly known as NaAMPS, which is available commercially at present from Lubrizol as either a 50% aqueous solution (reference code LZ2405) or a 58% aqueous solution (reference code LZ2405A); acrylic acid (3-sulphopropyl) ester potassium salt, commonly known as SPA or SPAK (SPA or SPAK is available commercially in the form of a pure solid from Raschig); acrylic acid (3-sulphopropyl) ester sodium salt, commonly known as SPANa (SPANa is available in the form of a pure solid from Raschig); N-acryloyl morpholine; and hydroxyethyl acrylamide.

The above monomers and monomer types may optionally include substituent groups.

Optional substituents of the monomers used to prepare the hydrogels used in the present invention may preferably be selected from substituents which are known in the art or are reasonably expected to provide polymerisable monomers which form hydrogel polymers having the properties necessary for the present invention. Suitable substituents include, for example, lower alkyl (e.g. C1 to C10, optionally C1 to C5), hydroxy, halo and amino groups.

### Cross-linking Agents

Conventional cross-linking agents are suitably used to provide the necessary mechanical stability and to control the adhesive properties of the hydrogel. The amount of cross-linking agent required will be readily apparent to those skilled in the art such as from about 0.01% to about 0.5%, particularly from about 0.05% to about 0.4%, most particularly from about 0.08% to about 0.3%, by weight of the total polymerisation reaction mixture. Typical crosslinkers include tripropylene glycol diacrylate, ethylene glycol dimethacrylate, triacrylate, polyethylene glycol diacrylate (polyethylene glycol (PEG) molecular weight between about 100 and about 4000, for example PEG400 or PEG600), and methylene bis acrylamide.

### Organic Plasticisers

The hydrogel and/or its pre-gel may comprise one or more organic plasticisers. The one or more organic plasticisers may suitably comprise any of the following either alone or in combination: at least one polyhydric alcohol (such as glycerol, polyethylene glycol, or sorbitol), at least one ester derived therefrom, at least one polymeric alcohol (such as polyethylene oxide) and/or at least one mono- or poly-alkylated derivative of a polyhydric or polymeric alcohol (such as alkylated polyethylene glycol). Glycerol is the preferred plasticiser. An alternative preferred plasticiser is the ester derived from boric acid and glycerol. When present, the organic plasticiser may comprise up to about 45% by weight of the hydrogel composition.

### Surfactants

Any compatible surfactant may optionally be used as an additional ingredient of the hydrogel composition. Surfactants can lower the surface tension of the mixture before polymerisation and thus aid processing. The surfactant or surfactants may be non-ionic, anionic, zwitterionic or cationic, alone or in any mixture or combination. The surfactant may itself be reactive, i.e. capable of participating in the hydrogel-forming reaction. The total amount of surfactant, if present, is suitably up to about 10% by weight of the hydrogel composition, preferably from about 0.05% to about 4% by weight.

In a preferred embodiment of the invention the surfactant comprises at least one propylene oxide/ethylene oxide block copolymer, for example such as that supplied by BASF Pic under the trade name Pluronic P65 or L64.

### Other additives

The hydrogel in the composite of the present invention may include one or more additional ingredients, which may be added to the pre-polymerisation mixture or the polymerised product, at the choice of the skilled worker. Such additional ingredients are selected from additives known in the art, including, for example, water, organic plasticisers, surfactants, polymeric material (hydrophobic or hydrophilic in nature, including proteins, enzymes, naturally occurring polymers and gums), synthetic polymers with and without- pendant carboxylic acids, electrolytes, pH regulators, colourants, chloride sources, bioactive compounds and mixtures thereof. The polymers can be natural polymers (e.g. xanthan gum), synthetic polymers (e.g. polyoxypropylene-polyoxyethylene block copolymer or poly-(methyl vinyl ether alt maleic anhydride)), or any combination thereof. By "bioactive compounds" we mean any compound or mixture included within the hydrogel for some effect it has on living systems, whether the living system be bacteria or other microorganisms or higher animals such as the patient. Bioactive compounds that may be mentioned include, for example, pharmaceutically active compounds, antimicrobial agents, antiseptic agents, antibiotics and any combination thereof. Antimicrobial agents may, for example, include: sources of oxygen and/or iodine (e.g. hydrogen peroxide or a source thereof and/or an iodide salt such as potassium iodide); honey (e.g. active Manuka honey); antimicrobial metals, metal ions and salts, such as, for example, silver-containing antimicrobial agents (e.g. colloidal silver, silver oxide, silver nitrate, silver thiosulphate, silver sulphadiazine, or any combination thereof); or any combination thereof.

Hydrogels incorporating antimicrobial agents may, for example, be active against such organisms as Staphylococcus aureus and Pseudomonas aeruginosa.

Additional polymer(s), typically rheology modifying polymer(s), may be incorporated into the polymerisation reaction mixture at levels typically up to about 10% by weight of total polymerisation reaction mixture, e.g. from about 0.2% to about 10% by weight.

Such polymer(s) may include polyacrylamide, poly-NaAMPS, polyethylene glycol (PEG), polyvinylpyrrolidone (PVP) or carboxymethyl cellulose.

The hydrogel used in the present invention may consists essentially of a cross-linked hydrophilic polymer of a hydrophilic monomer and optionally one or more comonomer, together with water and/or one or more organic plasticiser, and optionally together with one or more additives selected from surfactants, polymers, pH regulators, electrolytes, chloride sources, bioactive compounds and mixtures thereof, with less than about 30%, optionally less than about 20%, optionally less than 10% by weight of other additives.

The water activity, which is related to the osmolarity and the ionic strength of the precursor solution (as measured, for example, by a chilled mirror dewpoint meter, Aqualab T3) is preferably bewteen 0.05 and 0.99, more preferably between, 0.2 and 0.99, and even more preferably between 0.3 and 0.98. The ionic strength of the precursor solution can therefore be used to optimise the hydrogel composite properties.

For further details of the hydrogel material for use in the present invention, and its preparation, please refer to the following publications: PCT Patent Applications Nos. WO-97/24149, WO-97/34947, WO-00/06214, WO-00/06215, WO-00/07638, WO-00/46319, WO-00/65143, WO-01/96422 and WO 2007/007155.

The second material which is at least partially impregnated into the first material is a porous sheet comprising a non-woven material. The second material can be formed of a material that is natural in origin, synthetic in origin, or partly natural and partly synthetic. Typically the second material includes those formed from polyolefins, polyamides, polyacrylates, polyesters or combinations thereof such as polester/poyolefin bicomponent fibres.

The second material is a non-woven fabric having a low gram weight, of between 20 and 30 g/m².

The non-woven fabric can be corona-treated.

The second layer may be in the form of a sheet, which may be continuous or discontinuous, and disposed over part or all of the first layer.

The second layer, inclusive of an optional adhesive layer which may be disposed on a surface of the first layer, may have a thickness of from 25 - 60 microns. In a first preferred embodiment, the thickness of the second layer is 28 - 33 microns. In a second preferred embodiment the thickness of the second layer is 50 - 55 microns.

The second layer preferably comprises a low friction film material. Suitable film materials include polyurethanes, polyesters and polyolefins.

The low friction material is preferably such that clothing materials, such as cotton and/or polyester can pass easily over the surface of the material. A low friction material includes, but is not limited to, a material having a low coefficient of friction, which may be a static or dynamic coefficient of friction, for example against a cotton or Teflon material. Measurement of static and dynamic coefficients of friction are known to those skilled in the art. The static and dynamic coefficients of friction may be measured using standard equipment, and may be carried out at standard temperature and pressure, for example 20°C and 101.325 kPa. The static and dynamic coefficients of friction may be measured in accordance with ASTM 01894 - 08. Against a cotton material, the dynamic coefficient of friction of the second material may be about 1.5 or less, optionally about 1 or less, optionally about 0.9 or less. Against a Teflon material, the dynamic coefficient of friction of the second material may be about 1 or less, optionally about 0.7 or less, optionally about 0.5 or less, optionally about 0.4 or less.

The second layer may have a low friction value as measured in accordance with ISO 8295, 1986, in the test described in US 5,643,187 in column 11, lines 18 to 21. The friction may be measured as the dynamic friction according to ISO 8295, 1986, the base used being a teflon coated glass tissue of the type: Acoflon G 13 A from Aco-plast *NS,* Heisinger, DK, the weight of the carriage being 500 g. This test may be carried out at standard temperature and pressure, for example 20°C and 101.325 kPa. The frictional value of the third material, for example when in the form of a sheet, in N, when measured in accordance with this test may be about 5 or less, preferably about 3 or less, more preferably about 2 or less.

Under the same conditions, the force required to elongate the second layer in a lateral direction by 20% is preferably about 80% or less, more preferably about 70% or less and even more preferably about 50% or less, than that required to elongate the first layer by 20%. Under the same conditions, the force required to elongate the second layer in a lateral direction by 50% is preferably about 80% or less, more preferably about 70% or less and even more preferably about 50% or less, than that required to elongate the first layer by 50%. Under the same conditions, the force required to elongate the second layer in a lateral direction by 100% is preferably about 80% or less, more preferably about 70% or less and even more preferably about 50% or less, than that required to elongate the first layer by 100%.

The surface of the second layer which, in use, faces the wound is preferably coated with a pressure sensitive adhesive, for adhesion to one or more other layers with which it may be in contact and/or for adhesion to the skin. Suitable second layer materials coated with pressure sensitive adhesives include for example Dev-13-39A, Dev-13-41 A Inspire 2317 and Inspire 2327 from Exopack. The layered composition may also be termed a layered composite.

The composition can optionally be provided with an additional layer of the material of the second layer. This optional layer is located on the side of the composition which is in contact with a user's skin in use. The optional layer has the same dimensions as those of the second layer but is provided with an aperture to allow the first layer of hydrogel material to be placed against the skin when in use.

Typically the aperture has the same shape as the first layer. The aperture is typically sized such that at least 90% of the surface of the first layer is in contact with the skin in use.

The multilayer composition can optionally further comprise a release liner on the exposed lower surface of the multilayer dressing to facilitate ease of handling and packaging.

In an alternative embodiment the second material is fully impregnated in the first material.

According to a second aspect of the present invention there is provided a wound dressing comprising the multi-layered composition of the first aspect.

According to a third aspect of the present invention there is provided a composition of the first aspect for the prevention or treatment of a wound.

In the context of the present invention, the term 'wound' covers skin cuts, grazes, abrasions, tears, burns, scalds, ulcers, spots, blisters and chafes, whether dermal, epidermal, or a combination of both, and whether acute or chronic.

An embodiment of the present invention will now be described, by way of example only, with reference to the accompanying drawings in which:
Figure 1 illustrates a multi-layered composition in accordance with the present invention; and
Figure 2 illustrates an existing multi-layered composition.

Referring to Figure 1, a multi-layered composition in accordance with the present invention is generally shown at 1. The multi-layered composition comprises a first layer 2 (hereafter referred to as 'impregnated hydrogel') which itself comprises a hydrogel layer 3 which is partially impregnated with a 26 gsm PET non-woven fibre 4. A polyurethane film 5 is provided over the impregnated hydrogel 2 - the polyurethane film is located on the surface of the impregnated hydrogel 2 that is located away from the skin surface when in use. In the embodiment shown the surface of the impregnated hydrogel which is placed against the skin during use is provided with a layer of polyurethane film 6. The film 6 is provided with an aperture 7 that allows direct contact between the impregnated hydrogel layer 3 and the skin of a user (not shown). The polyurethane film 6 is provided with an adhesive to enable it to bind to the skin of a user when in use. The composition is also provided with a release liner (not shown) which is removed prior to use.

In contrast, Figure 2 a known multi-layered composition is generally shown at 8. The composition 8 includes a hydrogel layer 9 and a polyurethane layer 10. The composition 8 includes a polyester layer 12 which acts as a support to the polyurethane layer 9. The hydrogel layer 9 is provided with a PET layer 11 and layers of scrim material 13 and 14. The composition is also provided with a release liner 15 which is removed prior to use. Thus, this known composition requires significantly more structural complexity in order to be able to function.

The layers of the composition of the present invention can be constructed using techniques known to the man skilled in the art.

The resistance of the layered composition of the present application to lateral frictional loading was tested using a mechanism for applying a load.

### The compositions tested were as follows:

**Example 1**

| | |
|---|---|
| Upper Layer (Away from skin) | 30 micron Polyurethane film coated with pressure sensitive Acrylic adhesive |
| Shield Layer | 26 gsm polyester non woven |
| Cushioning Layer | 1100 gsm Hydrogel containing CarboxyMethylCellulose |

**Comparative example 1**

| | |
|---|---|
| Upper Layer (Away from skin) | 30 micron Polyurethane film coated with pressure sensitive Acrylic adhesive |
| Shield Layer | 100 micron PET |
| Cushioning Layer | 900 gsm Hydrogel containing CarboxyMethylCellulose |

**Comparative example 2**

| | |
|---|---|
| Upper Layer (Away from skin) | Polyurethane film |
| Cushioning Layer | A 3 dimensionally profiled hydrocolloid |

### Test Method:

All samples were tested using a Load Application Mechanism (LAM). The LAM is comprised of a rubbing head which has a curved anterior surface and a strip (60mm x 30mm x 0.9mm) of textured rubber material (Ironman Rubber Covering, Black, OB2090, Algeos UK ltd) providing an interface with the skin or dressing surface. This covering has a rough upper surface creating friction between the LAM device and skin and or dressing under test.

The rubbing head is attached to a level arm which is displaced vertically by the investigator. When the lever arm is displaced vertically it will contact two micro-switches causing compressed air to horizontally displace the rubbing head, bringing it into contact with the foot at a predetermined pressure, measured by a pressure sensor.

The samples under test were adhered to a plastic foot mode (Equipashop ID5476, female plastic foot display) and placed on the LAM device. The axial force under load was then adjusted to 7N. Load was applied and the time to failure recorded. Failure was defined as rupture of the backing film (and where occurring concurrently, the cushioning layer below).

### Results:

| Sample | Time to failure |
|---|---|
| Example 1 | 330s |
| Comparative example 1 | 90s |
| Comparative example 2 | 120s |

The results demonstrate that the use of a "soft" 26gsm shield layer give improved resistance failure from rupture of the upper film layer under lateral frictional loading of blister dressings using an in-vivo Load Application Mechanism vs. the use of either "hard" 100 micron PET shield layer or a Polyurethane covered hydrocolloid.

The cushioning properties of a composition of the present application with comparative example 1 were tested using a texture analyser

### Formulations:

**Example 1**

| | |
|---|---|
| Upper Layer (Away from skin) | 30 micron Polyurethane film coated with pressure sensitive Acrylic adhesive |
| Shield Layer | 26 gsm polyester non woven |
| Cushioning Layer | 1100 gsm Hydrogel containing CarboxyMethylCellulose |
| Average Sample thickness | 1085 microns |

**Comparative Example 1**

| | |
|---|---|
| Upper Layer (Away from skin) | 30 micron Polyurethane film coated with pressure sensitive Acrylic adhesive |
| Shield Layer | 100 micron PET |
| Cushioning Layer | 900 gsm Hydrogel containing CarboxyMethylCellulose |
| Average Sample thickness | 870 micron. |

### Test Method:

All samples were tested using a Stable Microsystems Texture Analyser TA Plus, fitted with a 1" Stainless Steel spherical probe. The insertion speed was set at 1mm/s and the retraction speed at 10 mm/s. The probe was inserted 2/3 of the sample thickness. Samples containing a shield layer were placed on the stage of the instrument with the shield layer facing downwards, such that only the cushioning directly attributed to the cushioning layer was measured. 4 probe insertions were performed for each sample point with the 1^{st} result of the series being discounted for all. Measurements were repeated in triplicate.

All Samples were pre-conditioned to the laboratory temperature and humidity for 24h prior to commencing the test. Measurements were taken in the centre of all dressing, i.e. the point of maximum thickness.

### Results:

| Sample | Cushioning value (g/mm) |
|---|---|
| Comparative Example 1 | 13175 |
| Example 1 | 30419 |

The results demonstrate that a 25% increase increase in thickness of the dressing gave an increase in cushioning of 130%, demonstrating the highly non linear cushioning behaviour of the composite structure.

An advantage of the present invention is that there is provided a composition having a simpler design over known multi-layer compositions which exhibits a significant reduction in the potential for wrinkles in the outer film to occur during application due to improved application. The non-woven material that is impregnated into the hydrogel provides improved flexibility over the PET layer in known compositions. This also results in improved adhesion of the composition of the present invention to a user's skin.

A further advantage of the present invention is that there is improved breathing at the site of application with a concomitant reduction in sweating.

When applied to a user's heel there is also a reduction in catching/snagging of the edge of the composition on a user's clothes or shoes.

## Claims

1. A multilayer composition comprising:
a first layer comprising a first material which is partially impregnated with a second material wherein the first material is a gel material and the second material is a flexible porous material, and
a second layer comprising a low friction material wherein the composition is adapted so that the first layer is in fluid flow communication with a wound or skin of a wearer in use,
wherein the gel in the first layer comprises a hydrogel, which in use contacts a wound, and
wherein the coat weight of the hydrogel is between 900 - 1400 g/m2; and
wherein the second material which is partially impregnated into the first material is a porous sheet comprises a non-woven material having a gram weight of between 20 and 30 g/m²; and
wherein the composition does not include an additional distinct/discrete layer between the first and second layers.

2. A composition as claimed in Claim 1 wherein the second material is formed of a material that is natural in origin, synthetic in origin, or partly natural and partly synthetic.

3. A composition as claimed in Claim 2 wherein the second material includes those formed from polyolefins, polyamides, polyacrylates, polyesters or combinations thereof such as polester/poyolefin bicomponent fibres; and/or wherein the non-woven material is corona-treated.

4. A composition as claimed in any of the preceding Claims wherein the second layer is in the form of a sheet, which may be continuous or discontinuous, and disposed over part or all of the first layer.

5. A composition as claimed in Claim 4 wherein the second layer, inclusive of an optional adhesive layer which may be disposed on a surface of the first layer, has a thickness of from 25 - 60 microns; and/or wherein the thickness of the second layer is 28 - 33 microns or 50 - 55 microns.

6. A composition as claimed in Claim 4 or Claim 5 wherein the second layer preferably comprises a low friction film material and optionally wherein the low friction film material is selected from polyurethanes, polyesters and polyolefins.

7. A composition as claimed in any of Claims 4 - 6 wherein the composition is provided with an additional layer of the material of the second layer and optionally wherein the optional additional layer is located on the side of the composition which is in contact with a user's skin in use and optionally wherein the optional additional layer has the same dimensions as those of the second layer but is provided with an aperture to allow the first layer of hydrogel material to be placed against the skin when in use.

8. A composition as claimed in Claim 7 wherein the aperture has the same shape as the first layer.

9. A composition as claimed in Claim 7 or Claim 8 wherein the aperture is typically sized such that at least 90% of the surface of the first layer is in contact with the skin in use.

10. A wound dressing comprising the multi-layered composition of Claims 1 - 9.

11. A composition as claimed in any of Claims 1 - 9 for the prevention or treatment of a wound.

12. A composition according to claim 11, wherein the wound is selected from skin cuts, grazes, abrasions, tears, burns, scalds, ulcers, spots, blisters and chafes.

13. A composition according to claim 11 or claim 12, wherein (i) the wound is dermal or epidermal or a combination of both and/or (ii) the wound is acute or chronic.

14. A composition according to claim 13, wherein the wound is a blister.

## Patentansprüche

1. Mehrschichtzusammensetzung, umfassend:
eine erste Schicht, umfassend ein erstes teilweise mit einem zweiten Material imprägniertes Material, wobei das erste Material ein Gel und das zweite Material ein flexibles und poröses Material ist und
eine zweite Schicht, die ein Material mit geringer Reibung umfasst, wobei die Zusammensetzung so ausgebildet ist, dass die erste Schicht im Gebrauch strömungstechnisch mit der Wunde oder der Haut des Trägers in Verbindung steht,
wobei das Gel in der ersten Schicht ein Hydrogel umfasst, das im Gebrauch eine Wunde berührt und
wobei das Strichgewicht des Hydrogels zwischen 900 und 1400 g/m² liegt; und
wobei das zweite teilweise in das erste Material imprägnierte Material eine poröse Folie ist, die ein ungewebtes Material mit einem Grammgewicht pro Quadratmeter zwischen 20 und 30 umfasst; und
wobei die Zusammensetzung zwischen der ersten und der zweiten Schicht keine zusätzliche separate/eigenständige Schicht aufweist.

2. Zusammensetzung gemäß Anspruch 1, wobei das zweite Material aus einem Material gebildet ist, das natürlichen Ursprungs, synthetischen Ursprungs oder teilweise natürlichen und teilweise synthetischen Ursprungs ist.

3. Zusammensetzung gemäß Anspruch 2, wobei das zweite Material diejenigen Materialien einschließt, die aus Polyolefinen, Polyamiden, Polyacrylaten, Polyestern oder Kombinationen davon gebildet sind, beispielsweise aus Polyester/Polyolefin-Bikomponentenfasern und/oder wobei das ungewebte Material mit Koronaentladungen behandelt wurde.

4. Zusammensetzung gemäß einem der vorhergehenden Ansprüche, wobei die zweite Schicht in Form einer kontinuierlichen oder diskontinuierlichen Folie vorliegt und ganz oder teilweise über der ersten Schicht angeordnet ist.

5. Zusammensetzung gemäß Anspruch 4, wobei die zweite Schicht gegebenenfalls eine auf einer Oberfläche der ersten Schicht angeordnete Haftschicht mit einer Stärke von 25 bis 60 µm aufweisen kann; und/oder wobei die Stärke der zweiten Schicht 28 bis 33 Mikrometer oder 50 bis 55 Mikrometer beträgt.

6. Zusammensetzung gemäß Anspruch 4 oder 5, wobei die zweite Schicht vorzugsweise eine reibungsarme Folie umfasst und die reibungsarme Folie gegebenenfalls aus Polyurethanen, Polyestern und/oder Polyolefinen gefertigt wurde.

7. Zusammensetzung gemäß einem der Ansprüche 4 bis 6, wobei die Zusammensetzung mit einer zusätzlichen Schicht des zweiten Schichtmaterials versehen ist und wobei sich die gegebenenfalls zusätzliche Schicht wahlweise auf der Seite der Zusammensetzung befindet, die im Gebrauch mit der Haut des Anwenders in Kontakt steht und wobei die gegebenenfalls zusätzliche Schicht die gleichen Abmessungen wie die zweite Schicht aufweist, jedoch mit einer Öffnung versehen ist, damit die erste Schicht aus Hydrogel im Gebrauch auf die Haut aufgelegt werden kann.

8. Zusammensetzung gemäß Anspruch 7, wobei die Öffnung die gleiche Form wie die erste Schicht hat.

9. Zusammensetzung gemäß Anspruch 7 oder Anspruch 8, wobei die Öffnung typischerweise so bemessen ist, dass im Gebrauch mindestens 90% der Oberfläche der ersten Schicht die Haut berühren.

10. Wundverband, umfassend die Mehrschichtzusammensetzung gemäß den Ansprüchen 1 bis 9.

11. Zusammensetzung gemäß einem der Ansprüche 1 bis 9 zur Vorbeugung oder Behandlung einer Wunde.

12. Zusammensetzung gemäß Anspruch 11, wobei die Wunde ein Schnitt in der Haut, eine Schürfwunde, eine Hautabschürfung, ein Riss in der Haut, eine Verbrennung, eine Verbrühung, eine Vereiterung, ein Pickel, eine Brandblase oder ein Hautwolf sein kann.

13. Zusammensetzung gemäß Anspruch 11 oder 12, wobei (i) die Wunde eine dermale oder epidermale Wunde oder eine Kombination von beiden und/oder (ii) die Wunde eine akute oder chronische Wunde sein kann.

14. Zusammensetzung gemäß Anspruch 13, wobei die Wunde eine Brandblase ist.

## Revendications

1. Une composition multicouche comprenant :
une première couche comprenant un premier matériau qui est partiellement imprégné d'un deuxième matériau dans laquelle le premier matériau est un matériau de gel et le deuxième matériau est un matériau poreux flexible, et
une deuxième couche comprenant un matériau à faible frottement dans laquelle la composition est adaptée de sorte que la première couche est en communication d'écoulement fluidique avec une plaie ou une peau d'un porteur en utilisation,
dans laquelle le gel dans la première couche comprend un hydrogel qui, en utilisation, entre en contact avec une plaie, et
dans laquelle le poids de revêtement de l'hydrogel est entre 900 et 1400 g/m² ; et
dans laquelle le deuxième matériau qui est partiellement imprégné dans le premier matériau est une feuille poreuse comprend un matériau non tissé ayant un poids en grammes entre 20 et 30 g/m² ; et
dans laquelle la composition ne comprend pas une couche distincte / discrète supplémentaire entre les première et deuxième couches.

2. Une composition selon la revendication 1, dans laquelle le deuxième matériau est formé d'un matériau qui est d'origine naturelle, d'origine synthétique, ou partiellement naturel et partiellement synthétique.

3. Une composition selon la revendication 2, dans laquelle le deuxième matériau comprend ceux formés à partir de polyoléfines, polyamides, polyacrylates, polyesters ou de combinaisons de ceux-ci tels que des fibres bicomposantes de polester / poyoléfine ; et / ou dans laquelle le matériau non tissé est traité par effet corona.

4. Une composition selon l'une quelconque des revendications précédentes, dans laquelle la deuxième couche est sous la forme d'une feuille, qui peut être continue ou discontinue, et disposée sur tout ou partie de la première couche.

5. Une composition selon la revendication 4, dans laquelle la deuxième couche, comprenant une couche adhésive facultative qui peut être disposée sur une surface de la première couche, a une épaisseur de 25 à 60 microns ; et /ou dans laquelle l'épaisseur de la deuxième couche est de 28 à 33 microns ou 50 à 55 microns.

6. Une composition selon la revendication 4 ou la revendication 5, dans laquelle la deuxième couche comprend de préférence un matériau de film à faible frottement et, facultativement, dans laquelle le matériau de film à faible frottement est choisi parmi les polyuréthannes, les polyesters et les polyoléfines.

7. Une composition selon l'une quelconque des revendications 4 à 6, dans laquelle la composition est munie d'une couche supplémentaire du matériau de la deuxième couche et facultativement dans laquelle la couche supplémentaire facultative est située sur le côté de la composition qui est en contact avec la peau d'un utilisateur en utilisation et facultativement dans laquelle la couche supplémentaire facultative a les mêmes dimensions que celles de la deuxième couche mais est munie d'une ouverture pour permettre à la première couche de matériau d'hydrogel d'être placée contre la peau en cours d'utilisation.

8. Une composition selon la revendication 7, dans laquelle l'ouverture a la même forme que la première couche.

9. Une composition selon la revendication 7 ou la revendication 8, dans laquelle l'ouverture est typiquement dimensionnée de telle sorte qu'au moins 90 % de la surface de la première couche soit en contact avec la peau en utilisation.

10. Un pansement pour plaie comprenant la composition multicouche selon les revendications 1 à 9.

11. Une composition selon l'une quelconque des revendications 1 à 9 pour la prévention ou le traitement d'une plaie.

12. Une composition selon la revendication 11, dans laquelle la plaie est choisie parmi les coupures cutanées, les éraflures, les abrasions, les déchirures, les brûlures, les échaudures, les ulcères, les boutons, les ampoules et les irritations.

13. Une composition selon la revendication 11 ou la revendication 12, dans laquelle (i) la plaie est dermique ou épidermique ou une combinaison des deux et / ou (ii) la plaie est aiguë ou chronique.

14. Une composition selon la revendication 13, dans laquelle la plaie est une ampoule.
